# EUROPÄISCHE PATENTSCHRIFT

(11) **EP 0 805 144 B1**
(45) Veröffentlichungstag und Bekanntmachung des Hinweises auf die Patenterteilung: **05.09.2001**
(21) Anmeldenummer: 97107328.3
(22) Anmeldetag: 02.05.1997
(51) Int. Cl.: C07C 213/02, C07C 215/12, C07C 215/24

(54) **Verfahren zur heterogen katalysierten Herstellung von hydroxy-alkyl-substituierten Aminoalkinen**
Process for the preparation of hydroxyalkyl substituted aminoalkynes through heterogeneous catalysis
Procédé de préparation d'aminoalkines hydroxyalkyle substituées par catalyse hétérogène

(30) Priorität: 03.05.1996 DE 19617802
(43) Veröffentlichungstag der Anmeldung: 05.11.1997
(73) Patentinhaber: BASF AKTIENGESELLSCHAFT, 67056 Ludwigshafen (DE)
(72) Erfinder: Rühl, Thomas, Dr., 67227 Frankenthal (DE); Preiss, Thomas, Dr., 67065 Ludwigshafen (DE); Henkelmann, Jochem, Dr., 68165 Mannheim (DE)
(74) Vertreter: Kinzebach, Werner, Dr.

(56) Entgegenhaltungen:
- EP-A- 0 080 794
- DE-A- 2 637 425
- US-A- 3 496 232
- JOURNAL OF GENERAL CHEMISTRY USSR, Bd. 31, 1961, NEW YORK US, Seiten 2127-32, XP002037075 N. M. LIBMAN ET AL.: "Amino alcohols of the acetylene series"
- CHEMICAL ABSTRACTS, vol. 66, no. 25, 19.Juni 1967 Columbus, Ohio, US; abstract no. 115346, VOLKOV, A. N. ET AL: "Synthesis and reactions of tertiary diacetylene- and alkoxyenynyl alicyclic alcohols" XP002037076 & ZH. ORG. KHIM. (1967), 3(2), 316-25,

## Beschreibung

Die vorliegende Erfindung betrifft ein Verfahren zur Herstellung von Aminoalkinen, die mindestens einen Hydroxyalkyl-Substituenten tragen.

Die Herstellung von nicht Hydroxyalkyl-substituierten Aminoalkinen ist seit langem bekannt und wird industriell genutzt. Dabei werden im allgemeinen entsprechend substituierte Alkine, Carbonylverbindungen und Amine in einer homogen oder heterogen katalysierten Reaktion im Sinne einer Mannich-Kondensation umgesetzt.

Homogen katalysierte Verfahren dieses Typs sind weit verbreitet und vielfach beschrieben. So beschreibt z.B. die CH-A-669 192 die Herstellung von pharmakologisch wirksamen N-Arylalkyl-substituierten Aminoalkinen in einer homogen katalysierten Reaktion, wobei als Katalysatoren Kupfer- und Zinksalze, wie z.B. CuCl oder ZnCl₂, eingesetzt werden.

Die US-3,496,232 beschreibt z.B. die Herstellung von N-Alkyl-substituierten Aminoalkinen in einer homogen oder heterogen katalysierten Mannich-Reaktion, wobei als Katalysatoren Salze von Metallen der ersten oder zweiten Nebengruppe, wie z.B. die Chloride, Acetate, Formiate und Acetylide und speziell Kupferacetylid eingesetzt werden, die im Falle der heterogen katalysierten Verfahrensvariante auf einem inerten Träger verwendet werden.

Die EP-A-0 080 794 beschreibt ein heterogen katalysiertes Verfahren zur Herstellung von N,N-disubstituierten Propinylaminen, wobei als bevorzugte Katalysatoren Kupferacetylide auf einem mit Bismutoxid dotierten Magnesiumsilikatträger eingesetzt werden. Die Reaktion erfolgt z.B. in einem Rührreaktor mit einem aufgeschlämmten Katalysator oder in einem Festbett, wobei Acetylen-Drücke von etwa 0,1 bis 20 atm auftreten.

In keiner der oben diskutierten Druckschriften findet der Fachmann einen Hinweis darauf, daß Hydroxyalkyl-substituierte Aminoalkine aus den Komponenten Alkin, Amin und Carbonylverbindung herstellbar sein könnten.

J. General Chem. USSR, 31, 1.961 (2127-32) beschreibt die Umsetzung von tertiären Propargylcarbinolen mit Formaldehyd und sekundären Aminen in Gegenwart von Cu₂C₂.

Die DE-A-26 37 425 beschreibt die Herstellung von Dialkylamino-2-alkin-4-olen durch Umsetzung von Formaldehyd, Dialkylamin und einem Alkinol. Um zufriedenstellende Ausbeuten zu erzielen, ist es jedoch erforderlich, spezielle Verfahrensbedingungen einzuhalten: Es muß in saurer Lösung, bevorzugt bei einem pH von 5, unter Verwendung eines speziellen Katalysatorsystems, nämlich einer Kombination von im Reaktionsgemisch löslichen Bromiden, Iodiden oder Jod und löslichen Cu(II)-Verbindungen gearbeitet werden. Die heterogen katalysierte Durchführung der Reaktion im neutralen oder alkalischen pH-Bereich wird nicht als mögliche Variante diskutiert. Diese Druckschrift enthält auch keine Hinweise darauf, daß N-Hydroxyalkyl-substituierte Aminoalkine herstellbar sind.

Dem Fachmann ist bewusst, daß bei der Herstellung von Aminoalkinen, die am Stickstoffatom und/oder am Alkin-Kohlenstoffatom Hydroxyalkyl-substituiert sind, mit einer ganzen Reihe von Nebenreaktionen zu rechnen ist, wenn man ein Gemisch aus Carbonylverbindung, Alkin und Amin, die gegebenenfalls Hydroxyalkyl-substituiert sind, umsetzt. Beim Einsatz von Mono- oder Di-Hydroxyalkyl-substituierten Aminen und von einer Carbonylverbindung ist mit Ringschlußreaktionen zu rechnen, wie z.B. der Oxazolidinbildung. Dies gilt insbesondere beim Einsatz von β-Aminoalkoholen als Aminkomponente, wenn unter alkalischen Reaktionsbedingungen in wasserfreien Lösungsmitteln oder in Gegenwart von wasserentziehenden Mitteln gearbeitet wird, und nicht, wie in der DE-A-26 37 425 beschrieben, in saurer, wässriger Lösung. Außerdem ist mit der Acetalbildung aus Hydroxyalkyl-substituierten Reaktanden und der Carbonylverbindung zu rechnen. Diese Acetale besitzen unter alkalischen Reaktionsbedingungen erhöhte Stabilität. Des weiteren kann es auch zur Bildung offenkettiger Kondensationsprodukte aus einem Hydroxyalkyl-substituierten Alkin, der Aminkomponente und einem entsprechenden Aldehyd kommen. Die Gefahr von Nebenreaktion ist insbesondere dann besonders groß, wenn die drei Reaktanden Amin, Alkin und Carbonylverbindung gleichzeitig eingesetzt werden.

Der vorliegenden Erfindung liegt deshalb die Aufgabe zugrunde, ein Verfahren zur Herstellung von Hydroxyalkyl-substituierten Aminoalkinen zur Verfügung zu stellen, das in hohen Ausbeuten zu den gewünschten Produkten führt und keine besonderen Maßnahmen zur Einstellung des pH-Werts erfordert.

Überraschenderweise wurde nun gefunden, daß diese Aufgabe gelöst wird durch Bereitstellung eines Verfahrens, bei dem ein Alkin mit einer Carbonylverbindung und einem Amin in einer heterogen katalysierten Reaktion umgesetzt wird, wobei mindestens einer der Substituenten am Aminstickstoff oder am nicht aminoalkylierten Alkinkohlenstoff ein Hydroxyalkylsubstituent ist. Überraschenderweise wurde außerdem festgestellt, daß keine besonderen Vorkehrungen zu pH-Wert-Einstellung zu treffen sind; es kann nämlich in dem durch die Reaktanden vorgegebenen neutralen bis alkalischen pH-Wert-Bereich gearbeitet werden, ohne daß es zu den erwarteten unerwünschten Nebenreaktionen kommt.

Gegenstand der vorliegenden Erfindung ist ein Verfahren zur Herstellung von Hydroxyalkyl-substituierten Aminoalkinen der allgemeinen Formel I 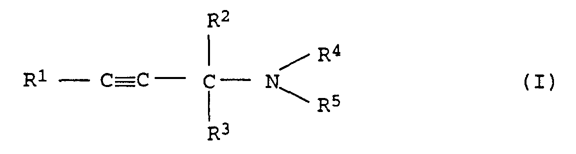 worin
- R¹: für ein Wasserstoffatom, Alkyl, Halogenalkyl, Cycloalkyl, Aryl, Alkoxy, Alkoxyalkyl oder Hydroxyalkyl steht;
- R² und R³: unabhängig voneinander für ein Wasserstoffatom, Alkyl, Halogenalkyl, Aryl oder Alkoxy stehen;
- R⁴ und R⁵: unabhängig voneinander für ein Wasserstoffatom, Alkyl, Halogenalkyl, Aryl, Alkoxy oder Hydroxyalkyl stehen, wobei R⁴ und R⁵ vorzugsweise nicht gleichzeitig für ein Wasserstoffatom stehen,oder
R⁴ und R⁵ zusammen mit dem Stickstoffatom, an das sie gebunden sind, einen 5- oder 6-gliedrigen heterocyclischen Ring bilden;
wobei mindestens einer der Substituenten R¹, R⁴ oder R⁵ für Hydroxyalkyl steht, das
dadurch gekennzeichnet ist, daß man ein Gemisch aus einem Alkin der allgemeinen Formel II

R¹― C≡C― H (II)

worin
R¹ die oben angegebenen Bedeutungen besitzt, einer Carbonylverbindung der allgemeinen Formel III 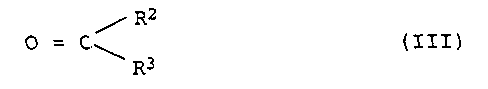 worin
R² und R³ die oben angegebenen Bedeutungen besitzen, und einem Amin der allgemeinen Formel IV 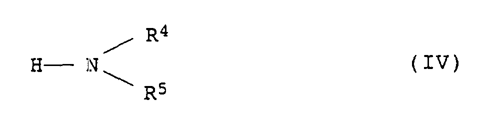 worin
R⁴ und R⁵ die oben angegebenen Bedeutungen besitzen,
   in einer heterogen katalysierten Reaktion umsetzt und als heterogener Katalysator eine Verbindung eines Metalls der ersten oder zweiten Nebengruppe auf einem inerten Träger verwendet wird, wobei die Umsetzung im neutralen oder alkalischen pH-Bereich erfolgt.

Im Rahmen der vorliegenden Erfindung steht Halogen für Fluor, Chlor, Brom und Jod und insbesondere für Chlor und Brom.

Der Ausdruck "Alkyl" umfasst geradkettige und verzweigte Alkylgruppen. Vorzugsweise handelt es sich dabei um geradkettige oder verzweigte C₁-C₁₂-Alkyl- und insbesondere C₁-C₆-Alkylgruppen. Beispiele für Alkylgruppen sind insbesondere Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 1-Ethylpentyl, 2-Ethylpentyl, 1-Propylbutyl, Octyl, Decyl und Dodecyl.

Halogenalkyl steht für eine wie oben definierte Alkylgruppe, die mit einem oder mehreren Halogenatomen, insbesondere Chlor und Brom, teilweise oder vollständig, vorzugsweise mit ein bis drei Halogenatomen, halogeniert ist.

Die obigen Ausführungen zur Alkylgruppe und Halogenalkylgruppe gelten in entsprechender Weise für die Alkylgruppe in Alkoxy-, Alkoxyalkyl- und Hydroxyalkyl-Resten.

Cycloalkyl steht vorzugsweise für C₃-C₈-Cycloalkyl, wie Cyclopropyl, Cyclobutyl, Cyclopentyl, Cyclohexyl, Cycloheptyl oder Cyclooctyl, oder Cyclopentylmethyl, Cyclopentylethyl und Cyclohexylmethyl und Cyclohexylethyl.

Aryl steht vorzugsweise für Phenyl oder Naphthyl.

Die Reste R⁴ und R⁵ können zusammen mit dem Stickstoffatom, an das sie gebunden sind, einen heterocyclischen Ring bilden. Beispiele hierfür sind Succinimido-, Phthalimido-Gruppen oder ein ungesättigter oder gesättigter 5- oder 6-gliedriger heterocyclischer Ring, der gegebenenfalls ein weiteres Heteroatom, ausgewählt unter S und N, vorzugsweise N, enthält. Als Beispiele können hierfür genannt werden: Piperidinyl-, Piperazinyl- und Tetrahydropyrimidinyl-Gruppen.

Gemäß einer bevorzugten Ausführungsform des erfindungsgemäßen Verfahrens werden Verbindungen hergestellt, worin der Rest R¹ für ein Wasserstoffatom, Alkyl oder C₁-C₆-Hydroxyalkyl steht.

Gemäß einer weiteren bevorzugten Ausführungsform des erfindungsgemäßen Verfahrens werden Verbindungen hergestellt, worin die Reste R² und R³ in der Formel III unabhängig voneinander für ein Wasserstoffatom oder Alkyl stehen. Insbesondere stehen die Reste R² und R³ beide für Wasserstoff.

Gemäß einer weiteren bevorzugten Ausführungsform des erfindungsgemäßen Verfahrens werden Verbindungen hergestellt, worin die Reste R⁴ und R⁵ unabhängig voneinander für ein Wasserstoffatom, Alkyl oder C₂-C₆-Hydroxyalkyl stehen.

Allgemein können für das erfindungsgemäße heterogen katalysierte Verfahren wie oben beschriebene Katalysatoren verwendet werden, wie sie auch für Ethinylierungsreaktionen verwendet werden. Solche Katalysatoren sind aus dem Stand der Technik bekannt und werden z.B. in der US-A-4,119,790 und in der EP-A-80 794 beschrieben. Im erfindungsgemäßen Verfahren wird eine Verbindung eines Metalls der ersten oder zweiten Nebengruppe des Periodensystems auf einem inerten Träger und insbesondere ein geträgerter Kupferacetylenidkatalysator verwendet, der aus einem Precursor hergestellt wird, welcher 10 bis 20 Gew.-% Kupferoxid, 1 bis 5 Gew.-% Bismutoxid und Siliciumdioxid als Trägermaterial umfasst. Besonders bevorzugt verwendet man einen Kupferacetylenid-Katalysator, der aus einem Percursor hergestellt wurde, der etwa 14 bis 15 Gew.-% CuO, etwa 4 Gew.-% Bi₂O₃ und etwa 80 % SiO₂ umfasst.

Die Reaktion kann lösungsmittelfrei oder in Gegenwart eines inerten organischen Lösungsmittels durchgeführt werden. Durch Zusatz eines wasserentziehenden Mittels, wie z.B. Natriumsulfat, lässt sich die Reaktion beschleunigen und die Ausbeute gegebenenfalls weiter erhöhen. Die Menge an eingesetzem wasserentziehenden Mittel liegt dabei bei bis zu 1 mol, je mol Carbonylverbindung gemäß der Formel III.

Die reaktionstechnische Ausführung des erfindungsgemäßen Verfahrens kann in einem Rühr-, Rohr- oder Schlaufenreaktor als kontinuierlicher oder diskontinuierlicher Prozess erfolgen. Die Reaktionstemperatur liegt im allgemeinen bei 40 bis 150°C, vorzugsweise bei etwa 60 bis 120°C.

Für niedrigsiedende substituierte Alkine und Amine wird die Reaktion unter Eigendruck durchgeführt, für höhersiedende Alkine und Amine erfolgt die Reaktion unter Normaldruck.

Ist die Verbindung der Formel II Acetylen, so wird die Carbonylverbindung der Formel III und das Amin der Formel IV gemeinsam mit dem Katalysator und gegebenenfalls einem Lösungsmittel in einem Autoklaven vorgelegt. Es wird Acetylen aufgepresst, bis ein Druck von etwa 3 bis 7 bar, wie z.B. etwa 5 bar erreicht ist, und der Autoklav anschliessend auf die Reaktionstemperatur erwärmt. Dann wird erneut Acetylen aufgepresst, bis ein konstanter Druck von etwa 15 bis 25 bar, z.B. etwa 20 bar erreicht ist.

Die Erfindung soll anhand des folgenden, nicht einschränkenden Beispiels erläutert werden.

### Beispiel 1

In einem 300 ml Rührautoklaven werden 22,5 g (0,75 mol) Paraformaldehyd und 78,8 g (0,75 mol) Diethanolamin mit 75 ml THF vorgelegt. Zu dieser Lösung wird dann 12 g aktivierter Kupferacetylenid-Katalysator (14-15 % CuO, 4 % Bi₂O₃, 80 % SiO₂) gegeben. Anschliessend werden 5 bar Acetylen aufgepresst und die Lösung auf 100°C erwärmt. Es wird dann mit Acetylen auf 20 bar nachgepresst, bis Druckkonstanz erreicht ist. Nach destillativer Aufarbeitung kann N,N'-Diethanol-3-aminopropin in 85 % Ausbeute isoliert werden.

Wie das Beispiel zeigt, werden durch das erfindungsgemäße Verfahren Aminoalkine der allgemeinen Formel I in einer heterogen katalysierten Reaktion in überraschend guten Ausbeuten zugängig gemacht.

### Beispiel 2

In einem 500 ml Dreihalskolben werden 112,12 g (2 mol) 2-Propin-1-ol mit 60 g Paraformaldehyd und 258,5 g (2 mol) Di-n-butylamin versetzt. Zu dieser Lösung werden 20 g aktivierter Kupferacetylenid-Katalysator (14 bis 15 % CuO, 4 % Bi₂O₃, 80 % SiO₂) gegeben. Die Mischung wird dann 12 Stunden bei 90°C gerüht. Durch destillative Aufarbeitung wird Di-n-butylamino-2-butin-1-ol in 82 % Ausbeute isoliert.

### Beispiel 3

In einem 500 ml Dreihalskolben werden 22,5 g (0,75 mol) Paraformaldehyd, 78,8 g (0,75 mol) Diethanolamin und 42 g (0,75 mol) Prop-1-in-3-ol mit 150 ml THF vorgelegt. Zu dieser Lösung wird dann 12 g aktivierter Kupferacetylenid-Katalysator (14 bis 15 % CuO, 4 % Bi₂O₃, 80 % SiO₂) gegeben. Anschliessend wird die Lösung auf 66°C erwärmt und 12 Stunden bei dieser Temperatur gerührt. Nach destillativer Aufarbeitung kann N,N'-Diethanol-4-aminobut-2-in-1-ol in 85 % Ausbeute isoliert werden.

## Patentansprüche

1. Verfahren zur Herstellung von Hydroxyalkyl-substituierten Aminoalkinen der allgemeinen Formel I 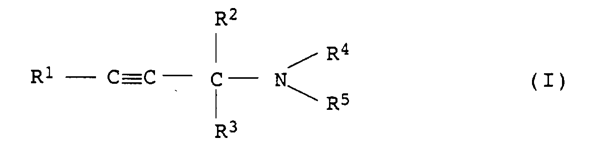 worin
R¹ für ein Wasserstoffatom, Alkyl, Halogenalkyl, Cycloalkyl, Aryl, Alkoxy, Alkoxyalkyl oder Hydroxyalkyl steht;
R² und R³ unabhängig voneinander für ein Wasserstoffatom, Alkyl, Halogenalkyl, Aryl oder Alkoxy stehen;
R⁴ und R⁵ unabhängig voneinander für ein Wasserstoffatom, Alkyl, Halogenalkyl, Aryl, Alkoxy oder Hydroxyalkyl stehen, oder R⁴ und R⁵ zusammen mit dem Stickstoffatom, an das sie gebunden sind, einen 5- oder 6-gliedrigen heterocyclischen Ring bilden;
wobei mindestens einer der Substituenten R¹, R⁴ oder R⁵ für Hydroxyalkyl steht,
bei dem man ein Gemisch aus
einem Alkin der allgemeinen Formel II
R¹―C≡C―H (II)
worin
R¹ die oben angegebenen Bedeutungen besitzt, einer Carbonylverbindung der allgemeinen Formel III 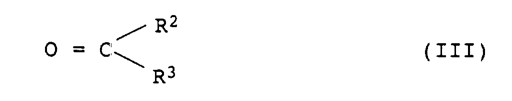 worin
R² und R³ die oben angegebenen Bedeutungen besitzen,
und einem Amin der allgemeinen Formel IV 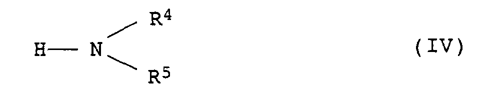 worin
R⁴ und R⁵ die oben angegebenen Bedeutungen besitzen, in einer heterogen katalysierten Reaktion umsetzt und als heterogener Katalysator eine Verbindung eines Metalls der ersten oder zweiten Nebengruppe auf einem inerten Träger verwendet wird, wobei die Umsetzung im neutralen oder alkalischen pH-Bereich erfolgt.

2. Verfahren nach einem der vorhergehenden Ansprüche, **dadurch gekennzeichnet**, daß als heterogener Katalysator ein Kupferacetylid-Katalysator auf einem mit Bismut dotierten Siliciumdioxid-Träger verwendet wird.

3. Verfahren nach einem der vorhergehenden Ansprüche, **dadurch gekennzeichnet**, daß bei der Reaktion ein wasserentziehendes Mittel in einer Menge von bis zu einem Mol je Mol eingesetzter Carbonylverbindung zugesetzt wird.

4. Verfahren nach einem der vorhergehenden Ansprüche, **dadurch gekennzeichnet**, daß die Reaktion in einem Rühr-, Rohr- oder Schlaufenreaktor kontinuierlich oder diskontinuierlich durchgeführt wird.

5. Verfahren nach einem der vorhergehenden Ansprüche, **dadurch gekennzeichnet**, daß die Umsetzung in nichtwässrigem Milieu erfolgt.

6. Verfahren nach einem der vorhergehenden Ansprüche, **dadurch gekennzeichnet**, daß man ein Alkin der Formel II umsetzt, worin R¹ für ein Wasserstoffatom, Alkyl oder Hydroxyalkyl steht.

7. Verfahren nach einem der vorhergehenden Ansprüche, **dadurch gekennzeichnet**, daß als Verbindung der Formel II Acetylen verwendet wird und die Reaktion in einem Autoklaven bei einem Acetyl en druck von bis zu etwa 20 bar durchgeführt wird.

8. Verfahren nach einem der Ansprüche 1 bis 6, **dadurch gekennzeichnet**, daß als Verbindung der Formel II ein substituiertes Alkin verwendet wird und die Reaktion unter Normaldruck oder dem Eigendruck der Reaktionsmischung durchgeführt wird.

9. Verfahren nach einem der vorhergehenden Ansprüche, **dadurch gekennzeichnet**, daß man eine Carbonylverbindung der Formel III umsetzt, worin R² und R³ unabhängig voneinander für ein Wasserstoffatom oder Alkyl stehen.

10. Verfahren nach einem der vorhergehenden Ansprüche, **dadurch gekennzeichnet**, daß man eine Carbonylverbindung der Formel III umsetzt, worin R² und R³ jeweils für ein Wasserstoffatom stehen.

11. Verfahren nach einem der vorhergehenden Ansprüche, **dadurch gekennzeichnet**, daß man ein Amin der Formel IV umsetzt, worin R⁴ und R⁵ unabhängig voneinander für ein Wasserstoffatom, Alkyl oder Hydroxyalkyl stehen.

12. Verfahren nach einem der vorhergehenden Ansprüche, **dadurch gekennzeichnet**, daß man ein Amin der Formel IV umsetzt, worin wenigstens einer der Reste R⁴ und R⁵ für Hydroxyalkyl steht.

## Claims

1. A process for preparing hydroxyalkyl-substituted aminoalkynes of the general formula I 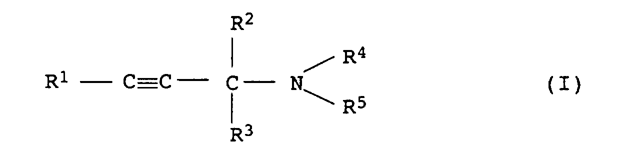 where
R¹ is hydrogen, alkyl, haloalkyl, cycloalkyl, aryl, alkoxy, alkoxyalkyl or hydroxyalkyl;
R² and R³ are, independently of one another, hydrogen, alkyl, haloalkyl, aryl or alkoxy;
R⁴ and R⁵ are, independently of one another, hydrogen, alkyl, haloalkyl, aryl, alkoxy or hydroxyalkyl, or R⁴ and R⁵ form, together with the nitrogen atom to which they are bonded, a 5- or 6-membered heterocyclic ring;
where at least one of the substituents R¹, R⁴ or R⁵ is hydroxyalkyl,
in which a mixture of
an alkyne of the general formula II
R¹― C≡C― H (II)
where
R¹ has the abovementioned meanings,
a carbonyl compound of the general formula III 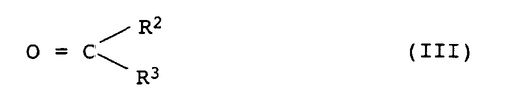 where
R² and R³ have the abovementioned meanings, and an amine of the general formula IV 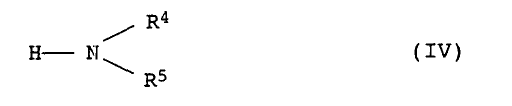 where
R⁴ and R⁵ have the abovementioned meanings, is reacted with heterogeneous catalysis, and a compound of a metal of group Ib or IIb on an inert carrier is used as heterogeneous catalyst, the reaction being carried out at neutral or alkaline pH.

2. A process as claimed in claim 1, wherein a copper acetylide catalyst on a silica carrier doped with bismuth is used as heterogeneous catalyst.

3. A process as claimed in either of the preceding claims, wherein a dehydrating agent is added in the reaction in an amount of up to one mol per mole of carbonyl compound employed.

4. A process as claimed in any of the preceding claims, wherein the reaction is carried out continuously or batchwise in a stirred, tubular or loop reactor.

5. A process as claimed in any of the preceding claims, wherein the reaction takes place in nonaqueous medium.

6. A process as claimed in any of the preceding claims, wherein an alkyne of the formula II where R¹ is hydrogen, alkyl or hydroxy alkyl is reacted.

7. A process as claimed in any of the preceding claims, wherein acetylene is used as compound of the formula II, and the reaction is carried out in an autoclave under an acetylene pressure of up to about 20 bar.

8. A process as claimed in any of claims 1 to 6, wherein a substituted alkyne is used as compound of the formula II, and the reaction is carried out under atmospheric pressure or the autogenous pressure of the reaction mixture.

9. A process as claimed in any of the preceding claims, wherein a carbonyl compound of the formula III where R² and R³ are, independently of one another, hydrogen or alkyl is reacted.

10. A process as claimed in any of the preceding claims, wherein a carbonyl compound of the formula III where R² and R³ are each hydrogen is reacted.

11. A process as claimed in any of the preceding claims, wherein an amine of the formula IV where R⁴ and R⁵ are, independently of one another, hydrogen, alkyl or hydroxyalkyl is reacted.

12. A process as claimed in any of the preceding claims, wherein an amine of the formula IV where at least one of the radicals R⁴ and R⁵ is hydroxyalkyl is reacted.

## Revendications

1. Procéde de préparation d'aminoalkines hydroxyalkyle substituées de la formule générale I 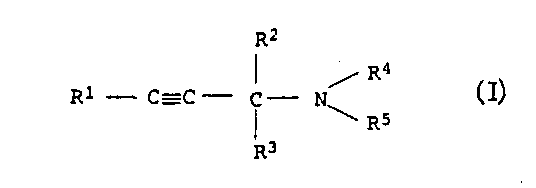 dans laquelle
R¹ représente un atome d'hydrogène ou un radical alkyle, halogénoalkyle, cycloalkyle, aryle, alcoxy, alcoxyalkyle ou hydroxyalkyle;
R² et R³ représentent indépendamment l'un de l'autre un atome d'hydrogène ou un radical alkyle, halogénoalkyle, aryle ou alcoxy;
R⁴ et R⁵ représentent indépendamment l'un de l'autre un atome d'hydrogène ou un radical alkyle, halogénoalkyle, aryle, alcoxy ou hydroxyalkyle, ou bien R⁴ et R⁵ forment ensemble avec l'atome d'azote auquel ils sont reliés un noyau hétérocyclique pentagonal à hexagonal;
où au moins l'un des substituants R¹, R⁴ ou R⁵ représentent un radical hydroxyalkyle,
dans lequel on fait réagir un mélange de:
une alkine de la formule générale II
R¹-C ≡ C-H (II)
où
R¹ a la signification ci-dessus,
un composé carbonyle de la formule générale III 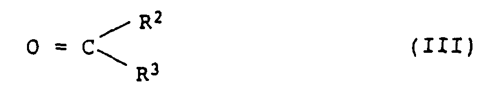 où
R² et R³ ont les significations ci-dessus,
et une amine de la formule générale IV 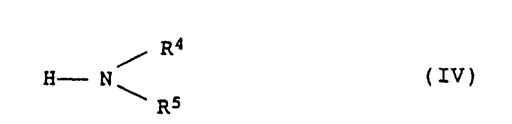 où
R⁴ et R⁵ ont les significations ci-dessus,
par une réaction à catalyse hétérogène, et où l'on utilise comme catalyseur hétérogène un composé d'un métal du premier ou du deuxième sous-groupe sur un support inerte, la réaction étant entreprise dans une plage de pH neutre ou alcaline.

2. Procédé selon la revendication qui précède, **caractérisé en ce que** l'on utilise comme catalyseur hétérogène un catalyseur d'acétylure de cuivre sur un support de dioxyde de silicium dopé au bismuth.

3. Procédé selon l'une des revendications qui précédent, **caractérisé en ce que**, lors de la réaction, l'on ajoute un agent déshydratant en quantité jusqu'à une mole par mole de composé carbonyle mis en oeuvre.

4. Procédé selon l'une des revendications qui précèdent, **caractérisé en ce que** la réaction est entreprise, en mode discontinu ou continu, dans un réacteur à brassage, un réacteur tubulaire, ou une colonne à loulles à écoulement en boucle.

5. Procédé selon l'une des revendications qui précèdent, **caractérisé en ce que** la réaction est entreprise en milieu non aqueux.

6. Procédé selon l'une des revendications qui précèdent, **caractérisé en ce que** l'on fait réagir une alkine de la formule II où R¹ représente un atome d'hydrogène ou un radical alkyle ou hydroxyalkyle.

7. Procédé selon l'une des revendications qui précèdent, **caractérisé en ce que** l'on utilise de l'acétylène en tant que composé de la formule II et que la réaction est entreprise dans un autoclave sous une pression d'acétylène jusqu'à environ 20 bar.

8. Procédé selon l'une des revendications 1 à 6, **caractérisé en ce que** l'on utilise comme composé de la formule II une alkine substituée et que la réaction est entreprise sous pression normale ou à la pression propre du mélange réactionnel.

9. Procédé selon l'une des revendications qui précèdent, **caractérisé en ce que** l'on fait réagir un composé carbonyle de la formule III dans lequel R² et R³ représentent, indépendamment l'un de l'autre, un atome d'hydrogène ou un radical alkyle.

10. Procédé selon l'une des revendications qui précèdent, **caractérisé en ce que** l'on fait réagir un composé carbonyle de la formule III dans lequel R² et R³ représentent à chaque fois un atome d'hydrogène.

11. Procédé selon l'une des revendications qui précèdent, **caractérisé en ce que** l'on fait réagir une amine de la formule IV dans laquelle R⁴ et R⁵ représentent, indépendamment l'un de l'autre, un atome d'hydrogène ou un radical alkyle ou hydroxyalkyle.

12. Procédé selon l'une des revendications qui précèdent, **caractérisé en ce que** l'on fait réagir une amine de la formule IV dans laquelle les restes R⁴ et R⁵ représentent un radical hydroxyalkyle.
